(19) Europäisches Patentamt
European Patent Office
Office européen des brevets

(11) EP 4 407 063 A1

(12) **EUROPEAN PATENT APPLICATION**
published in accordance with Art. 153(4) EPC

(43) Date of publication:
**31.07.2024 Bulletin 2024/31**

(21) Application number: **22895377.4**

(22) Date of filing: **26.10.2022**

(51) International Patent Classification (IPC):
***C23C 2/02*** *(2006.01)* ***G01N 23/223*** *(2006.01)*
***G01N 33/2028*** *(2019.01)*

(52) Cooperative Patent Classification (CPC):
**C23C 2/02; G01N 23/223; G01N 33/2028**

(86) International application number:
**PCT/JP2022/039864**

(87) International publication number:
**WO 2023/090093 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.11.2021 JP 2021188119**
**14.09.2022 JP 2022146404**

(71) Applicant: **KABUSHIKI KAISHA KOBE SEIKO SHO (KOBE STEEL, LTD.)**
**Hyogo 651-8585 (JP)**

(72) Inventors:
- **SASAKI Koji**
  **Kakogawa-shi, Hyogo 675-0137 (JP)**
- **KOBAYASHI Masanori**
  **Kakogawa-shi, Hyogo 675-0137 (JP)**
- **FUKUSHIMA Koki**
  **Kakogawa-shi, Hyogo 675-0137 (JP)**
- **HAYASHI Kazushi**
  **Kakogawa-shi, Hyogo 675-0137 (JP)**
- **INUI Masahiro**
  **Kakogawa-shi, Hyogo 675-0137 (JP)**

(74) Representative: **Müller-Boré & Partner Patentanwälte PartG mbB**
**Friedenheimer Brücke 21**
**80639 München (DE)**

(54) **METHOD FOR CALCULATING FILM THICKNESS OF GRAIN BOUNDARY OXIDE LAYER, METHOD FOR DETERMINING PLATABILITY, METHOD FOR PRODUCING PLATED STEEL SHEET, AND APPARATUS FOR CALCULATING FILM THICKNESS**

(57) An aspect of the present invention is a method for calculating a film thickness of a grain boundary oxide layer remaining on a steel sheet after a pickling treatment, the method including: an intensity-measuring step in which a fluorescent X-ray intensity of an element being contained in the steel sheet and constituting an oxide in the grain boundary oxide layer is measured; and a film thickness-calculating step in which a film thickness of the grain boundary oxide layer is calculated based on: a correlation between a pre-extracted fluorescent X-ray intensity and the film thickness of the grain boundary oxide layer; and the fluorescent X-ray intensity measured in the intensity-measuring step.

FIG. 1

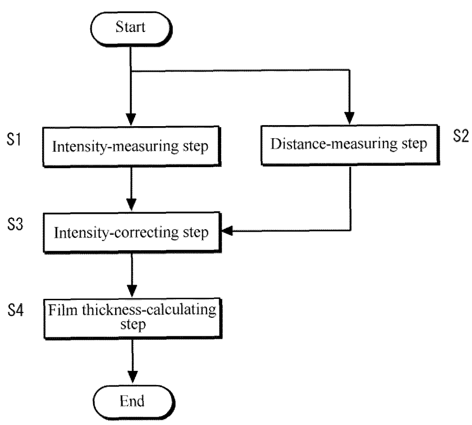


EP 4 407 063 A1

## Description

[TECHNICAL FIELD]

The present invention relates to a method for calculating a film thickness of a grain boundary oxide layer, a method for determining plating properties, a method for manufacturing a plated steel sheet, and a film thickness calculation device.

[BACKGROUND ART]

As high-strength steel sheets for use in car bodies and the like, surface-treated steel sheets imparted with a rust-preventive property, particularly a hot-dip galvanized steel sheet and a hot-dip galvannealed steel sheet, which are superior in rust prevention, are known. In general, a hot-dip galvanized steel sheet is manufactured by: using a belt-shaped steel sheet obtained by hot-rolling and cold-rolling a slab as a base steel sheet; recrystallizing and annealing the base steel sheet in a reducing atmosphere in an annealing furnace; and then subjecting the base steel sheet to a hot-dip galvanization process.

To increase a strength of such a high-strength steel sheet, addition of Si, Mn, and/or the like is effective. However, in the case in which the steel sheet contains Si, Mn, and/or the like, oxidation proceeds even in a reducing atmosphere containing a hydrogen gas, which is reductive and does not cause oxidation of iron, and an oxide of Si, Mn, and/or the like is formed on a surface of the steel sheet. This oxide deteriorates the wettability between molten zinc and the steel sheet in a plating process; thus, in a case of using a base steel sheet to which Si, Mn, and/or the like is added, plating properties are likely to deteriorate.

As a method for improving the plating properties of the base steel sheet to which Si, Mn, and/or the like is added, a manufacturing method by an oxidation-reduction method using an annealing furnace having an oxidation zone and a reduction zone is in practical use. In this manufacturing method, an oxide film of iron is formed on a surface of a steel sheet, the oxide film is reduced in a reducing atmosphere containing hydrogen, and then a plating process is performed.

It is known that a grain boundary oxide layer has an influence on the formation of the oxide film by the oxidation-reduction method. The grain boundary oxide layer is a layer in which an oxide is formed in such a manner that solid solution elements in Fe move to grain boundaries and are bonded to oxygen dispersed along the grain boundaries. A thickness of the grain boundary oxide layer can be adjusted by conditions for pickling preceding the oxidation-reduction process.

For example, in a case in which the grain boundary oxide layer is thin, i.e., an amount of the solid solution elements is large, Si, Mn, and/or the like being less noble than Fe is selectively oxidized during the oxidation-reduction annealing; therefore, the formation of the oxide film may be inhibited, leading to non-plating or uneven alloying. On the other hand, when the grain boundary oxide layer is thick, for example, the alloying may be excessively advanced, and ensuring the plating properties may not be possible. Thus, the thickness of the grain boundary oxide layer is an important factor for ensuring the plating properties.

As a method for specifying, in an in-line manner, an amount of the grain boundary oxide layer, the amount being considered to correlate with the thickness of the grain boundary oxide layer, a method for specifying an oxygen amount per unit area of the steel sheet, i.e., an oxygen coating amount by Compton scattering radiation utilizing X-rays has been proposed (see Japanese Unexamined Patent Application, Publication No. 2001-272360). In this specifying method, by utilizing the fact that a Compton scattering intensity increases with an increase in the oxygen amount due to oxidation of a surface layer, the oxygen coating amount is unambiguously obtained from an actually measured scattering intensity. Furthermore, based on a premise that the oxygen amount is proportional to the amount of the grain boundary oxide layer, the oxygen amount is used as an indicator for the amount of the grain boundary oxide layer.

[PRIOR ART DOCUMENTS]

[PATENT DOCUMENTS]

Patent Document 1: Japanese Unexamined Patent Application, Publication No. 2001-272360

[SUMMARY OF THE INVENTION]

[PROBLEMS TO BE SOLVED BY THE INVENTION]

The above conventional specifying method involves the premise that the oxygen amount is proportional to the amount of the grain boundary oxide layer. To satisfy this premise, it is necessary that a constitution of the oxide contained in the grain boundary oxide layer does not change. Here, as described above, the thickness of the grain boundary oxide layer before the formation of the oxide film by the oxidation-reduction method is adjusted by the pickling conditions. Specifically, a part of the grain boundary oxide layer which is thick is dissolved by the pickling to be thinned to a desired thickness and is allowed to remain. At this time, a specific oxide is selectively dissolved in the acid, resulting in the constitution of the oxide contained in the grain boundary oxide layer changing before and after the pickling, and also changing depending on the amount of the grain boundary oxide layer dissolved by the pickling.

Accordingly, with regard to the film thickness of the grain boundary oxide layer remaining on the steel sheet after the pickling treatment, the premise of the above conventional specifying method that the oxygen amount is proportional to the amount of the grain boundary oxide layer is not satisfied in the first place, making it difficult to accurately specify the film thickness of the grain boundary oxide layer. Therefore, it is difficult to accurately evaluate the plating properties of the steel sheet by the method using the oxygen amount.

The present invention was made in view of the foregoing circumstances, and an object of the present invention is to provide: a method for calculating a film thickness of a grain boundary oxide layer and a film thickness calculation device, the method and the film thickness calculation device enabling accurately calculating the film thickness of the grain boundary oxide layer remaining on the steel sheet after the pickling treatment; a method for determining plating properties by which the plating properties of the steel sheet are accurately determined from the grain boundary oxide layer remaining on the steel sheet after the pickling treatment; and a method for manufacturing a plated steel sheet, the method using the method for determining plating properties.

[MEANS FOR SOLVING THE PROBLEMS]

In the steel sheet before the pickling treatment, a relatively thick grain boundary oxide layer is formed on a surface of a steel sheet base. At this stage, a concentration of an element being contained in the steel sheet and constituting the oxide in the grain boundary oxide layer is uniform between the steel sheet base and an inside of the grain boundary oxide layer. When the steel sheet is subjected to the pickling treatment, the oxide of the above element is selectively dissolved, whereby the concentration of the element in the grain boundary oxide layer is decreased. When a fluorescent X-ray intensity is measured with respect to the above element of the steel sheet, the intensity is determined by the above element in the vicinity of the surface, and thus, the fluorescent X-ray intensity is lower than that in a case of measuring the steel sheet base. Moreover, the present inventors have focused on the fact that when the pickling treatment is continued and the thickness of the grain boundary oxide layer remaining approaches a desired value, the grain boundary oxide layer becomes sufficiently thin, and thus, the intensity derived from the above element contained in the steel sheet base is superimposed on the fluorescent X-ray intensity. In other words, the present inventors have found that in this region, a proportion of the fluorescent X-rays superimposed due to the above element contained in the steel sheet base increases as the grain boundary oxide layer becomes thinner, thereby increasing the fluorescent X-ray intensity to be measured. Furthermore, the present inventors have found that the fluorescent X-ray intensity has a high correlation with the film thickness of the grain boundary oxide layer, thereby completing the present invention.

That is to say, one aspect of the present invention is a method for calculating a film thickness of a grain boundary oxide layer remaining on a steel sheet after a pickling treatment, the method including: an intensity-measuring step in which a fluorescent X-ray intensity of an element being contained in the steel sheet and constituting an oxide in the grain boundary oxide layer is measured; and a film thickness-calculating step in which a film thickness of the grain boundary oxide layer is calculated based on: a correlation between a pre-extracted fluorescent X-ray intensity and the film thickness of the grain boundary oxide layer; and the fluorescent X-ray intensity measured in the intensity-measuring step.

In the method for calculating a film thickness of a grain boundary oxide layer, the fluorescent X-ray intensity of the element being contained in the steel sheet and constituting the oxide in the grain boundary oxide layer is measured. Since the fluorescent X-ray intensity has a high correlation with the film thickness of the grain boundary oxide layer remaining on the steel sheet after the pickling treatment, the film thickness can be accurately calculated based on the fluorescent X-ray intensity.

It is preferable that a plurality of correlations are pre-extracted in accordance with manufacturing conditions of the steel sheet, and that in the film thickness-calculating step, one correlation is selected from the plurality of correlations in accordance with the manufacturing conditions of the steel sheet. For example, the fluorescent X-ray intensity depends on a Mn concentration. In the case in which the manufacturing conditions of the steel sheet are thus varied, the correlation may be varied. Therefore, by enabling selecting an appropriate correlation in accordance with the manufacturing conditions of the steel sheet, the calculation accuracy of the film thickness can be further improved.

The method preferably further includes: a distance-measuring step in which a measurement distance is measured, the measurement distance being a distance between the steel sheet and a measurement position of the fluorescent X-ray intensity measured in the intensity-measuring step; and an intensity-correcting step in which the fluorescent X-ray intensity measured in the intensity-measuring step is corrected based on the measurement distance, wherein in the film thickness-calculating step, the fluorescent X-ray intensity corrected in the intensity-correcting step is used instead of the fluorescent X-ray intensity measured in the intensity-measuring step. The fluorescent X-ray intensity is influenced by the measurement distance. Therefore, a decrease in the calculation accuracy of the film thickness can be inhibited by normalizing the fluorescent X-ray intensity by the measurement distance.

It is preferable that an acid used in the pickling treatment is hydrochloric acid, and that the element is Mn. By thus using hydrochloric acid as the acid in the pickling treatment and using Mn as the element, a measurement sensitivity of the fluorescent X-ray intensity can be improved, whereby the calculation accuracy of the film thickness can be further increased.

Another aspect of the present invention is a method for determining plating properties of a steel sheet from a grain boundary oxide layer remaining on the steel sheet after a pickling treatment, the method including: a calculating step of calculating a film thickness of the grain boundary oxide layer; and a determining step of determining plating properties of the steel sheet by using, as a determination indicator, the film thickness of the grain boundary oxide layer calculated in the calculating step, wherein the method for calculating a film thickness of a grain boundary oxide layer of the present invention is used in the calculating step.

In the method for determining plating properties, the film thickness of the grain boundary oxide layer can be accurately calculated by the method for calculating a film thickness of a grain boundary oxide layer of the present invention. Accordingly, the plating properties of the steel sheet can be accurately determined based on the film thickness calculated.

Still another aspect of the present invention is a method for determining plating properties of a steel sheet from a grain boundary oxide layer remaining on the steel sheet after a pickling treatment, the method including: an intensity-measuring step in which a fluorescent X-ray intensity of an element being contained in the steel sheet and constituting an oxide in the grain boundary oxide layer is measured; and a determining step in which plating properties of the steel sheet are directly determined based on the fluorescent X-ray intensity measured in the intensity-measuring step.

In the method for determining plating properties, the fluorescent X-ray intensity of the element being contained in the steel sheet and constituting the oxide in the grain boundary oxide layer is measured. Since the fluorescent X-ray intensity has a high correlation with the film thickness of the grain boundary oxide layer remaining on the steel sheet after the pickling treatment, the plating properties of the steel sheet can be directly and accurately calculated based on the fluorescent X-ray intensity.

A method for manufacturing a plated steel sheet according to another aspect of the present invention includes: a hot-rolling step in which a steel sheet serving as a base material is hot-rolled; a pickling step in which an oxide scale on a surface of the steel sheet after the hot-rolling step is removed by an acid; and a plating property-determining step in which plating properties of the steel sheet are determined from a grain boundary oxide layer remaining on the steel sheet after the pickling step, wherein the method for determining plating properties of the present invention is used in the plating property-determining step.

In the method for manufacturing a plated steel sheet, by using the method for determining plating properties of the present invention after the pickling step, the plating properties of the steel sheet can be accurately determined. Thus, the manufacturing conditions after the pickling step can be optimized in accordance with the plating properties of the steel sheet. Accordingly, by using the method for manufacturing a plated steel sheet, a manufacturing efficiency of the plated steel sheet can be increased.

Still another aspect of the present invention is a film thickness calculation device for measuring a film thickness of a grain boundary oxide layer remaining on a steel sheet after a pickling treatment, the film thickness calculation device including: an intensity measurement portion for measuring a fluorescent X-ray intensity of an element being contained in the steel sheet and constituting an oxide in the grain boundary oxide layer; a distance measurement portion for measuring a measurement distance being a distance between the steel sheet and a measurement position of the fluorescent X-ray intensity of the intensity measurement portion; an intensity correction portion for correcting, based on the measurement distance, the fluorescent X-ray intensity measured by the intensity measurement portion; and a film thickness calculation portion for calculating a film thickness of the grain boundary oxide layer based on: a correlation between a pre-extracted fluorescent X-ray intensity and the film thickness of the grain boundary oxide layer; and the fluorescent X-ray intensity corrected by the intensity correction portion.

The film thickness calculation device includes the intensity measurement portion for measuring the fluorescent X-ray intensity of the element being contained in the steel sheet and constituting the oxide in the grain boundary oxide layer. Since the fluorescent X-ray intensity has a high correlation with the film thickness of the grain boundary oxide layer formed on the steel sheet after the pickling treatment, the film thickness calculation portion of the film thickness calculation device can accurately calculate the film thickness of the grain boundary oxide layer based on the fluorescent X-ray intensity. Furthermore, the film thickness calculation device enables inhibiting a decrease in the calculation accuracy by making use of the measurement distance to normalize the fluorescent X-ray intensity, which is influenced by the measurement distance. Accordingly, the film thickness calculation device can accurately calculate the film thickness of the grain boundary oxide layer.

The film thickness calculation device preferably further includes a storage portion for storing a plurality of correlations extracted in accordance with manufacturing conditions of the steel sheet, wherein the film thickness calculation portion has a function of selecting one correlation from the plurality of correlations in accordance with the manufacturing conditions of the steel sheet. By thus enabling selecting an appropriate correlation in accordance with the manufacturing conditions of the steel sheet, the calculation accuracy of the film thickness can be further improved.

[EFFECTS OF THE INVENTION]

As described above, according to the method for calculating a film thickness of a grain boundary oxide layer and the film thickness calculation device of the present invention, the film thickness of the grain boundary oxide layer remaining on the steel sheet after the pickling treatment can be accurately calculated. According to the method for determining plating properties of the present invention, the plating properties of the steel sheet can be accurately determined from the grain boundary oxide layer remaining on the steel sheet after the pickling treatment. Furthermore, the method for manufacturing a plated steel sheet of the present invention includes the method for determining plating properties, which enables the plating properties of the steel sheet to be accurately determined from the grain boundary oxide layer, and thus, the manufacturing efficiency can be improved.

[BRIEF DESCRIPTION OF THE DRAWINGS]

FIG. 1 is a flowchart illustrating a method for calculating a film thickness according to one embodiment of the present invention.
FIG. 2 is a flowchart illustrating a method for determining plating properties according to one embodiment of the present invention.
FIG. 3 is a flowchart illustrating a method for manufacturing a plated steel sheet according to one embodiment of the present invention.
FIG. 4 is a schematic configuration diagram of a film thickness calculation device according to one embodiment of the present invention, a plating property determination device using the film thickness calculation device, and a plated steel sheet-manufacturing device using the plating property determination device.
FIG. 5 is a flowchart illustrating an extraction method for extracting the correlation between the fluorescent X-ray intensity and the film thickness of the grain boundary oxide layer, the correlation being used in the film thickness-calculating step in FIG. 1.
FIG. 6 is a schematic configuration diagram of a film thickness calculation device according to an embodiment different from that of the film thickness calculation device in FIG. 4.
FIG. 7 is a graph for explaining a correlation between a relative intensity of the fluorescent X-rays and the thickness of the grain boundary oxide layer.
FIG. 8 is a flowchart illustrating a method for determining plating properties according to an embodiment different from that in FIG. 2.
FIG. 9 is a graph showing a relation between pickling treatment time and a decrease in mass due to the pickling treatment in EXAMPLES.
FIG. 10 is a graph showing a correlation between the fluorescent X-ray intensity and the film thickness of the grain boundary oxide layer in EXAMPLES.
FIG. 11 is a graph showing a correlation between actually measured values and the film thickness of the grain boundary oxide layer calculated according to the determination method in FIG. 4 in EXAMPLES.

[DESCRIPTION OF EMBODIMENTS]

First Embodiment

Hereinafter, the method for calculating a film thickness of a grain boundary oxide layer, the method for determining plating properties, the method for manufacturing a plated steel sheet, and the film thickness calculation device according to the one embodiment of the present invention are described.
The method for calculating a film thickness of a grain boundary oxide layer of the present invention is a method for calculating a film thickness of a grain boundary oxide layer remaining on a steel sheet after a pickling treatment and includes, as illustrated in FIG. 1, an intensity-measuring step S1, a distance-measuring step S2, an intensity-correcting step S3, and a film thickness-calculating step S4. The method for calculating a film thickness of a grain boundary oxide layer is used in the method for determining plating properties which itself is the one embodiment of the present invention.

Specifically, the method for determining plating properties is a method for determining plating properties of a steel sheet from a grain boundary oxide layer remaining on the steel sheet after a pickling treatment and includes, as illustrated in FIG. 2, a calculating step S5 and a determining step S6, wherein the method for calculating a film thickness of a grain boundary oxide layer is used in the calculating step S5. The method for determining plating properties is used in the method for manufacturing a plated steel sheet which itself is the one embodiment of the present invention. Specifically, the method for manufacturing a plated steel sheet includes, as illustrated in FIG. 3, a hot-rolling step S11, a pickling step S12, a plating property-determining step S13, and a cold-rolling step S14, wherein the method for determining plating properties is used in the plating property-determining step S13.

The method for calculating a film thickness of a grain boundary oxide layer can be carried out by a film thickness calculation device 1 illustrated in FIG. 4, which itself is the one embodiment of the present invention. The film thickness calculation device 1 is used in a plating property determination device 2 illustrated in FIG. 4, and the plating property determination device 2 is used in a plated steel sheet-manufacturing device 100 illustrated in FIG. 4. The plated steel sheet-manufacturing device 100 includes: a hot-rolling portion 110 for hot-rolling a steel sheet M serving as a base material; a pickling portion 120 for removing, by an acid, an oxide scale on a surface of the steel sheet M hot-rolled by the hot-rolling portion 110; and a cold-rolling portion 130 for cold-rolling the steel sheet M from which the oxide scale has been removed by the pickling portion 120. The film thickness calculation device 1 and accordingly, the plating property determination device 2 including the same are provided on a downstream side of the pickling portion 120 and an upstream side of the cold-rolling portion 130. In other words, the steel sheet M to be measured by an intensity measurement portion 10 described later of the film thickness calculation device 1 has been hot-rolled but has not been cold-rolled.

The film thickness calculation device 1 is a film thickness calculation device for calculating a film thickness of a grain boundary oxide layer remaining on the steel sheet M after a pickling treatment, specifically, for calculating the film thickness (a calculated film thickness 1a) of the grain boundary oxide layer remaining on the steel sheet M after the pickling treatment. The film thickness calculation device 1 includes: the intensity measurement portion 10 for measuring a fluorescent X-ray intensity of an element being contained in the steel sheet M and constituting an oxide in the grain boundary oxide layer; a distance measurement portion 20 for measuring a measurement distance L being a distance between the steel sheet M and a measurement position of the fluorescent X-ray intensity of the intensity measurement portion 10; an intensity correction portion 30 for correcting, based on the measurement distance L, the fluorescent X-ray intensity measured by the intensity measurement portion 10; and a film thickness calculation portion 40 for calculating the film thickness of the grain boundary oxide layer based on: a correlation between a pre-extracted fluorescent X-ray intensity and the film thickness of the grain boundary oxide layer; and the fluorescent X-ray intensity corrected by the intensity correction portion 30.

The plating property determination device 2 includes, in addition to the film thickness calculation device 1, a determination portion 50 for determining plating properties of the steel sheet M by using, as a determination indicator, the film thickness (calculated film thickness 1a) of the grain boundary oxide layer calculated by the film thickness calculation portion 40.

## Method for Manufacturing Plated Steel Sheet

Hereinafter, each step of the method for manufacturing a plated steel sheet is described.

### Hot-Rolling Step

In the hot-rolling step S11, the steel sheet M serving as a base material is hot-rolled. The hot-rolling step S11 is performed by the hot-rolling portion 110.

Specifically, the following procedure is performed. For example, from a cast coil C1 obtained by rolling up steel such as a cast slab or the like into a coil shape and cooling the steel once to room temperature, the steel sheet M is reeled out in a sheet passing direction R and charged into the hot-rolling portion 110. It is to be noted that the steel such as a cast slab or the like may be directly charged into the hot-rolling portion 110.

The steel sheet M charged into the hot-rolling portion 110 is soaked and hot-rolled. Rolling conditions are not particularly limited. The steel sheet M hot-rolled has a high temperature of, for example, greater than or equal to 500 °C and is rolled up on a reel. An uncooled hot-rolled coil C2 after the rolling up is cooled (for example, air-cooled) to a normal temperature.

### Pickling Step

In the pickling step S12, the oxide scale on the surface of the steel sheet M after the hot-rolling step S11 is removed by the acid (pickling treatment). The pickling step S12 is performed by the pickling portion 120.

Specifically, the following procedure is performed. From a cooled hot-rolled coil C3 having the normal temperature, the steel sheet M is reeled out in the sheet passing direction R and charged into the pickling portion 120. The pickling portion 120 includes, for example, a pickling tank retaining the acid, and a surface oxide film formed in the hot-rolling step S11 is dissolved and removed by immersing the steel sheet M in the pickling tank. At this time, in light of the plating properties, the grain boundary oxide layer is allowed to remain on the surface of the steel sheet M. An average thickness of the grain boundary oxide layer to be allowed to remain is preferably greater than or equal to 5 $\mu$m and less than or equal to 20 $\mu$m. As referred to herein, the "average thickness" means an average of thicknesses measured at ten arbitrary points.

The acid used in the pickling treatment can be exemplified by hydrochloric acid, sulfuric acid, and the like and is preferably hydrochloric acid. By thus using hydrochloric acid as the acid in the pickling treatment, an extraction sensitivity in the calculating step S5 of the plating property-determining step S13 described later, i.e., in the method for calculating a film thickness, can be improved, and thus, the extraction accuracy of the film thickness of the grain boundary oxide layer can be further increased.

Plating Property-Determining Step

In the plating property-determining step S13, the plating properties of the steel sheet M are determined from the grain boundary oxide layer remaining on the steel sheet M after the pickling step S12. Specifically, the film thickness of the grain boundary oxide layer remaining on the steel sheet M after the pickling step S12 is extracted to determine the plating properties thereof.

In the plating property-determining step S13, the method for determining plating properties illustrated in FIG. 2 is used. The details of the method for determining plating properties are described later.

As a film thickness calculation position, a position at which the steel sheet M after the pickling is tensioned by a bridle roll or the like to inhibit sheet vibrations is preferred. Furthermore, a calculation result is preferably stored for each coil.

When the calculated film thickness of the grain boundary oxide layer is used, grouping of the steel sheet M is enabled in accordance with the film thickness of the grain boundary oxide layer. By thus grouping in accordance with the film thickness of the grain boundary oxide layer, for example, it is possible to manufacture plated steel sheets with reduced original sheet variations. Furthermore, a steel sheet M in which defective plating may occur owing to the original sheet can be predicted, and thus, a countermeasure such as disposal or the like of the steel sheet M can be carried out as needed. Moreover, a cause for the defective plating can be isolated into a factor of the original sheet and that of the manufacturing conditions, and thus, the manufacturing conditions can be easily optimized.

The plating property-determining step S13 is preferably continuously performed in a longitudinal direction (the sheet passing direction R) of the steel sheet M. By thus continuously performing the plating property-determining step S13 along the longitudinal direction of the steel sheet M, in a case in which there arises a region in which the film thickness of the grain boundary oxide layer is partly thin, the steel sheet M can be used by cutting off this region.

In the method for manufacturing a plated steel sheet, the pickling step S12 is performed before the cold-rolling step S14. In the plating property-determining step S13 after the pickling step S12 and before the cold-rolling step S14, the film thickness of the grain boundary oxide layer formed on the steel sheet M after the pickling treatment is extracted, whereby the manufacturing conditions in and after the cold-rolling step S14, being the next step, can be optimized in accordance with the film thickness of the grain boundary oxide layer. Accordingly, by using the method for manufacturing a plated steel sheet, the manufacturing efficiency of the plated steel sheet can be increased.

Cold-Rolling Step

In the cold-rolling step S14, the steel sheet M after the plating property-determining step S13 is cold-rolled. The cold-rolling step S14 is performed by the cold-rolling portion 130.

Specifically, the cold rolling can be performed by putting the steel sheet M during passing, between rolling rolls. As the rolling rolls of the cold-rolling portion 130, a configuration in which a plurality of pairs of cold-rolling rolls as illustrated in FIG. 4 are arranged may be employed, or a reverse mill in which rolling is repeatedly performed by one mill may be used.

The steel sheet M rolled is rolled up on a reel to form a coil (cold-rolled coil C4) of the cold-rolled steel sheet.

The cold-rolled coil C4 is subjected to a conventional process, i.e., an oxidizing step of oxidizing the surface of the steel sheet M, a reducing step of reducing an oxide film formed in the oxidizing step, and a plating step of plating the surface of the steel sheet M after the reducing step, whereby the plated steel sheet is obtained.

Method for Determining Plating Properties

Next, each step of the method for determining plating properties used in the plating property-determining step S13 of the above-described method for manufacturing a plated steel sheet is described.

Calculating Step

In the calculating step S5, the film thickness of the grain boundary oxide layer is calculated. As described above, the method for calculating a film thickness of a grain boundary oxide layer illustrated in FIG. 1 is used in the calculating step S5. Hereinafter, as the calculating step S5, each step of the method for calculating a film thickness of a grain boundary oxide layer is described.

Intensity-Measuring Step

In the intensity-measuring step S1, the fluorescent X-ray intensity of the element being contained in the steel sheet M and constituting the oxide in the grain boundary oxide layer is measured.

The intensity-measuring step S1 is performed by the intensity measurement portion 10. As the intensity measurement portion 10, a known fluorescent X-ray analyzer may be used.

As the above element, an element which is contained in the steel sheet M, an oxide of which is selectively removed by pickling, is selected. In particular, it is preferable that the above element is Mn, and it is more preferable that the acid used in the pickling treatment is hydrochloric acid and the above element is Mn. The oxide to be selectively removed and a removal percentage thereof are determined by a combination of a type of a pickling solution and a type of the element. The removal percentage is high in the case of the combination in which the acid used in the pickling treatment is hydrochloric acid and the above element is Mn. In the method for calculating a film thickness of a grain boundary oxide layer, the measurement sensitivity is improved as the removal percentage becomes higher; therefore, the above combination enables further increasing the calculation accuracy of the film thickness and accordingly, the determination accuracy in the method for determining plating properties.

Distance-Measuring Step

In the distance-measuring step S2, the measurement distance L, being the distance between the steel sheet M and the measurement position of the fluorescent X-ray intensity measured in the intensity-measuring step S 1, is measured.

The distance-measuring step S2 is performed by the distance measurement portion 20. As the distance measurement portion 20, for example, a known laser displacement meter may be used.

Intensity-Correcting Step

In the intensity-correcting step S3, the fluorescent X-ray intensity measured in the intensity-measuring step S1 is corrected based on the measurement distance L.

The intensity-correcting step S3 is performed by the intensity correction portion 30. As the intensity correction portion 30, a known arithmetic device such as a microcontroller, a personal computer, or the like may be used.

Even if a fluorescent X-ray analyzer at a time of extracting a correlation described later is used and the intensity-measuring step S 1 is performed using an equivalent measurement distance $L_0$, the actual measurement distance L may have an error with respect to the measurement distance $L_0$ at the time of extracting the correlation. Since the fluorescent X-ray intensity is influenced by the measurement distance L, this error may directly lead to an error of the film thickness to be calculated. In the intensity-correcting step S3, the measurement distance L is actually measured, and the fluorescent X-ray intensity is normalized by the above measurement distance in relation to the measurement distance $L_0$ at the time of extracting the correlation.

Even in the case in which the measurement distance L, which is actually measured, is thus different from the measurement distance $L_0$ at the time of extracting the correlation between the fluorescent X-ray intensity and the film thickness of the grain boundary oxide layer, by including the distance-measuring step S2 and the intensity-correcting step S3 and by normalizing the fluorescent X-ray intensity by the measurement distance, a decrease in the determination accuracy can be inhibited.

For example, the fluorescent X-ray intensity decreases in inverse proportion to the square of the measurement distance L, and thus, when the fluorescent X-ray intensity measured in the intensity-measuring step S1 is denoted by A and a corrected fluorescent X-ray intensity is denoted by Ac, the fluorescent X-ray intensity can be corrected according to the following equation 1.

$$Ac = A \times (L/L_0)^2 \qquad 1$$

Alternatively, with regard to an actually expected range of the measurement distance L, the fluorescent X-ray intensity may be actually measured in advance, a database of a relation between the measurement distance L and the corrected fluorescent X-ray intensity Ac may be made, for example, in a lookup table form, and a corrected value may be determined based on the database.

Film Thickness-Calculating Step

In the film thickness-calculating step S4, the film thickness of the grain boundary oxide layer is calculated based on: the correlation between the pre-extracted fluorescent X-ray intensity and the film thickness of the grain boundary oxide layer; and the fluorescent X-ray intensity measured in the intensity-measuring step.

The film thickness-calculating step S4 is performed by the film thickness calculation portion 40. As the film thickness calculation portion 40, a known arithmetic device such as a microcontroller, a personal computer, or the like may be used. The arithmetic device of the intensity correction portion 30 may be shared with the film thickness calculation portion 40.

Here, the method for extracting the correlation between the fluorescent X-ray intensity and the film thickness of the grain boundary oxide layer is described. The correlation can be extracted by subjecting a plurality of steel sheet samples on which grain boundary oxide layers with different film thicknesses remain, to an extraction method including, as illustrated in FIG. 5, an individual X-ray intensity-measuring step S21, a breakpoint-extracting step S22, an individual film thickness-calculating step S23, and a correlation equation-determining step S24.

Individual X-ray Intensity-Measuring Step

In the individual X-ray intensity-measuring step S21, the fluorescent X-ray intensity of the above element of an individual steel sheet sample is measured.

This step is preferably performed using the same device as in the intensity measurement portion 10 used in the intensity-measuring step S1, with the measurement distance L being equivalent. By thus including the same measurement conditions as the intensity-measuring step S1, errors can be reduced.

Breakpoint-Extracting Step

In the breakpoint-extracting step S22, a breakpoint of a polyline representing a relation between the pickling treatment time of the steel sheet sample measured in the individual X-ray intensity-measuring step S21 and a decrease in mass per unit area due to the pickling treatment is obtained.

With regard to each individual steel sheet sample, when considering the relation between the pickling treatment time and the decrease in mass per unit area due to the pickling treatment, the mass decreases owing to the removal of the grain boundary oxide layer for a certain period of time after the start of the pickling treatment. When the grain boundary oxide layer is eventually completely removed, a base metal of the steel sheet then begins to be removed. Since the grain boundary oxide layer and the base metal of the steel sheet have different compositions, the decrease in mass decreased per unit time due to dissolution by the pickling treatment, i.e., a mass decrease rate varies. Therefore, with regard to each individual steel sheet sample, the relation between the pickling treatment time and the decrease in mass due to the pickling treatment is represented by a polyline (for example, see FIG. 9 of the Examples). This breakpoint means a boundary between the grain boundary oxide layer and the base metal of the steel sheet. This breakpoint can be obtained, for example, by approximating the relation between the pickling treatment time and the decrease in mass due to the pickling treatment, the relation being discretely plotted, by a polyline broken at one point.

Individual Film Thickness-Calculating Step

In the individual film thickness-calculating step S23, a converted film thickness of the grain boundary oxide layer of the steel sheet sample is calculated by dividing the decrease in mass at the breakpoint by a density of the steel sheet.

Since the breakpoint represents a point at which the grain boundary oxide layer is completely removed but the steel sheet base is not yet removed, the decrease in mass per unit area at the breakpoint represents a total amount per unit area of the grain boundary oxide layer remaining on the steel sheet sample. A density of the grain boundary oxide layer is substantially equal to the density of the steel sheet; therefore, when the decrease in mass at the breakpoint is divided by the density of the steel sheet, for example, by 7.87 g/cm$^3$ of iron, the film thickness of the grain boundary oxide layer of the steel sheet sample can be accurately obtained.

Correlation Equation-Determining Step

In the correlation equation-determining step S24, a correlation equation between the fluorescent X-ray intensity and the film thickness of the grain boundary oxide layer is extracted from the fluorescent X-ray intensity and the converted film thickness of the grain boundary oxide layer of each individual steel sheet sample, which have been obtained in the individual X-ray intensity-measuring step S21 and the individual film thickness-calculating step S23.

As described above, the grain boundary oxide layer is relatively thin, and thus, when the fluorescent X-ray intensity of the above element of the steel sheet M is measured, an intensity derived from the above element contained in the steel sheet base is superimposed on the measured fluorescent X-ray intensity. Furthermore, as the grain boundary oxide layer becomes thinner, a proportion of the superimposition of the intensity derived from the above element contained in the steel sheet base increases. Here, since the oxide containing the above element has been selectively removed from the grain boundary oxide layer, the concentration of the above element in the grain boundary oxide layer is lower than the concentration of the above element contained in the steel sheet base. Accordingly, since the fluorescent X-ray intensity increases as the grain boundary oxide layer becomes thinner, the fluorescent X-ray intensity and the film thickness of the grain boundary oxide layer have a negative correlation (for example, see FIG. 10 of the Examples).

In the correlation equation-determining step S24, this correlation is represented by a correlation equation. As the correlation equation, for example, a linear expression is preferably used. A desired value exists for the film thickness of the grain boundary oxide layer, and the pickling treatment conditions are determined such that the desired value can be obtained; therefore, it is difficult to imagine that significant variations occur, and sufficient accuracy can be obtained with the linear approximation. As the linear approximation, for example, a known least squares method may be used.

By thus determining the correlation equation between the film thickness of the grain boundary oxide layer obtained in the individual film thickness-calculating step S23 and the fluorescent X-ray intensity of the steel sheet sample obtained in the individual X-ray intensity-measuring step S21, the film thickness (calculated film thickness 1a) of the grain boundary oxide layer can be easily and accurately calculated based on the fluorescent X-ray intensity corrected in the intensity-correcting step S3.

Determining Step

In the determining step S6, the plating properties of the steel sheet M are determined using, as a determination indicator, the film thickness of the grain boundary oxide layer calculated in the calculating step S5. Specifically, whether the thickness of the grain boundary oxide layer remaining has the desired value is determined. Upon determination, a case in which the film thickness is not exactly equal to the desired value but falls within a certain range may be determined as the plating properties being favorable.

The determining step S6 is performed by the determination portion 50. As the determination portion 50, a known arithmetic device such as a microcontroller, a personal computer, or the like may be used. The arithmetic device of the intensity correction portion 30 or the film thickness-calculating step S4 may be shared with the determination portion 50.

Advantages

In the method for calculating a film thickness of a grain boundary oxide layer, the fluorescent X-ray intensity of the element being contained in the steel sheet M and constituting the oxide in the grain boundary oxide layer is measured. Since the fluorescent X-ray intensity has a high correlation with the film thickness of the grain boundary oxide layer remaining on the steel sheet M after the pickling treatment, the film thickness can be accurately calculated based on the fluorescent X-ray intensity.

In the method for determining plating properties, the film thickness of the grain boundary oxide layer can be accurately calculated by the method for calculating a film thickness of a grain boundary oxide layer of the present invention. Accordingly, the plating properties of the steel sheet M can be accurately determined based on the calculated film thickness.

In the method for manufacturing a plated steel sheet, by using the method for determining plating properties of the present invention after the pickling step S12, the plating properties of the steel sheet M can be accurately determined. Thus, the manufacturing conditions after the pickling step S12 can be optimized in accordance with the plating properties of the steel sheet M. Accordingly, by using the method for manufacturing a plated steel sheet, the manufacturing efficiency of the plated steel sheet can be increased.

The film thickness calculation device 1 includes the intensity measurement portion 10 for measuring the fluorescent X-ray intensity of the element being contained in the steel sheet M and constituting the oxide in the grain boundary oxide layer. Since the fluorescent X-ray intensity has a high correlation with the film thickness of the grain boundary oxide layer formed on the steel sheet M after the pickling treatment, the film thickness calculation portion 40 of the film thickness calculation device 1 can accurately calculate the film thickness of the grain boundary oxide layer based on the fluorescent X-ray intensity. Furthermore, the film thickness calculation device 1 enables inhibiting a decrease in the calculation accuracy by making use of the measurement distance L to normalize the fluorescent X-ray intensity, which is influenced by the measurement distance L. Accordingly, the film thickness calculation device 1 can accurately calculate the film thickness of the grain boundary oxide layer.

Second Embodiment

Hereinafter, a method for calculating a film thickness of a grain boundary oxide layer and a film thickness calculation device according to a second embodiment of the present invention are described.

Film Thickness Calculation Device

A film thickness calculation device 3 illustrated in FIG. 6 is a film thickness calculation device for calculating the film thickness of the grain boundary oxide layer remaining on the steel sheet M after the pickling treatment. The film thickness calculation device 3 can be used instead of the film thickness calculation device 1 illustrated in FIG. 4.

The film thickness calculation device 3 includes: the intensity measurement portion 10 for measuring the fluorescent X-ray intensity of the element being contained in the steel sheet M and constituting the oxide in the grain boundary oxide layer; the distance measurement portion 20 for measuring the measurement distance L being the distance between the steel sheet M and the measurement position of the fluorescent X-ray intensity of the intensity measurement portion 10; the intensity correction portion 30 for correcting, based on the measurement distance L, the fluorescent X-ray intensity measured by the intensity measurement portion 10; and a film thickness calculation portion 41 for calculating the film thickness (calculated film thickness 1a) of the grain boundary oxide layer based on: the correlation between the pre-extracted fluorescent X-ray intensity and the film thickness of the grain boundary oxide layer; and the fluorescent X-ray intensity corrected by the intensity correction portion 30. Furthermore, the film thickness calculation device 3 further includes a storage portion 60.

The intensity measurement portion 10, the distance measurement portion 20, and the intensity correction portion 30 may have configurations similar to those of the intensity measurement portion 10, the distance measurement portion 20, and the intensity correction portion 30 in FIG. 4; thus, the same reference symbols are assigned to these portions, and detailed description thereof is omitted.

Storage Portion

The storage portion 60 stores a plurality of correlations extracted in accordance with manufacturing conditions of the steel sheet M.

Examples of the manufacturing conditions of the steel sheet M include the acid used in the pickling treatment, a type and a concentration of an element contained in the steel sheet M, and the like. Of these, the Mn concentration is preferred as the manufacturing condition of the steel sheet M. It is known that the fluorescent X-ray intensity depends on the Mn concentration.

AMn concentration dependency of the fluorescent X-ray intensity is described in detail; in a case in which single-wavelength X-rays are incident on the steel sheet M having a two-layer structure of the steel sheet base and the grain boundary oxide layer, assuming that the Mn concentrations in the steel sheet base and the grain boundary oxide layer are constant, the relative intensity of the fluorescent X-rays derived from the Mn in the steel sheet M is represented by the following equation 2. Here, $W_1$ denotes the Mn concentration (% by mass) in the grain boundary oxide layer, $W_2$ denotes the Mn concentration (% by mass) in the steel sheet base, $T_1$ denotes the film thickness (cm) of the grain boundary oxide layer, $\rho_1$ denotes the density (g/cm$^3$) of the grain boundary oxide layer, and A is a constant represented by the following equation 3. Const. is a constant determined by an element to be measured and an optical system of the X-rays. Furthermore, in the following equation 3, $\mu_1(\lambda)$ denotes a mass absorption coefficient (cm$^2$/g) of the incident X-rays in the grain boundary oxide layer, $\mu_1$(ip) denotes a mass absorption coefficient (cm$^2$/g) of the fluorescent X-rays in the grain boundary oxide layer, $\Phi$ denotes an incident angle (rad) of the incident X-rays, and $\Psi$ denotes a detection angle (rad) of the fluorescent X-rays.

$$I = \{W_1\{1 - \exp(-A\rho_1 T_1)\} + W_2 \exp(-A\rho_1 T_1)\} \times Const. \quad \cdots 2$$

$$A = \frac{\mu_1(\lambda)}{sin\Phi} + \frac{\mu_1(ip)}{sin\psi} \quad \cdots 3$$

A main component of the grain boundary oxide layer is Fe, and thus, assuming that the mass absorption coefficients in the grain boundary oxide layer do not change, A is considered constant even with variations in the Mn concentration and/or the like. In other words, the fluorescent X-ray intensity I serves as functions of $W_1$, $W_2$, and $T_1$.

Now, given that a Mn concentration of 2.0% by mass is set as a designed value of the steel sheet M, it is assumed that the Mn concentration is changed to 1.8% by mass or 2.2% by mass owing to variations in steelmaking. Furthermore, when it is assumed that the amount of a Mn oxide formed in the grain boundary oxide layer is constantly 0.5% by mass and that the Mn oxide is completely removed in the pickling step, the Mn concentration $W_1$ in the grain boundary oxide layer after the pickling is 1.3% by mass to 1.7% by mass. The graph in FIG. 7 is obtained by calculating, according to the above equation by using these numerical values, the correlation between the relative intensity of the fluorescent X-rays and the thickness of the grain boundary oxide layer. Here, in the calculation, the incident X-rays are a Rh Ka-line, the fluorescent X-rays are a Mn Ka-line, the incident angle of the incident X-rays is 60°, the detection angle is 90°, and the density of the grain boundary oxide layer is 7.87 g/cm$^3$. Furthermore, the results are normalized by setting the fluorescent X-ray intensity of the grain boundary oxide layer having a film thickness of 0 $\mu$m in a case in which the Mn concentration in the grain boundary oxide layer is 1.5% by mass and the Mn concentration in the steel sheet base is 2.0% by mass, to 1.

It can be seen from the graph in FIG. 7 that even when the same fluorescent X-ray intensity is obtained, a change in the Mn concentration in the steel sheet base may cause a change in the film thickness of the grain boundary oxide layer. Therefore, in the film thickness calculation device 3, the plurality of correlations extracted in accordance with the manufacturing conditions (in this case, the Mn concentration in the steel sheet base) of the steel sheet M are prepared.

The number of correlations to be stored is not particularly limited. The number may be determined as appropriate in accordance with the manufacturing conditions of the steel sheet M and/or corresponding differences in correlations.

Film Thickness Calculation Portion

The film thickness calculation portion 41 has a function of selecting one correlation from the plurality of correlations in accordance with the manufacturing conditions of the steel sheet M. By thus enabling selecting an appropriate correlation in accordance with the manufacturing conditions of the steel sheet M, the calculation accuracy of the film thickness can be further improved.

Except for having the above function, the film thickness calculation portion 41 may have a configuration similar to that of the film thickness calculation portion 40 illustrated in FIG. 4, and thus, detailed description thereof is omitted.

Method for Calculating Film Thickness of Grain Boundary Oxide Layer

The method for calculating a film thickness of a grain boundary oxide layer, the method using the film thickness calculation device 3 illustrated in FIG. 6, is a method for calculating the film thickness of the grain boundary oxide layer remaining on the steel sheet M after the pickling treatment and includes: an intensity-measuring step in which the fluorescent X-ray intensity of the element being contained in the steel sheet M and constituting the oxide in the grain boundary oxide layer; a distance-measuring step in which the measurement distance L being the distance between the steel sheet M and the measurement position of the fluorescent X-ray intensity measured in the intensity-measuring step is measured; an intensity-correcting step in which the fluorescent X-ray intensity measured in the intensity-measuring step is corrected based on the measurement distance L; and a film thickness-calculating step in which the film thickness of the grain boundary oxide layer is calculated, based on: the correlation between the pre-extracted fluorescent X-ray intensity and the film thickness of the grain boundary oxide layer; and the fluorescent X-ray intensity measured in the intensity-correcting step.

The intensity-measuring step, the distance-measuring step, and the intensity-correcting step are similar to the intensity-measuring step S 1, the distance-measuring step S2, and the intensity-correcting step S3 of the first embodiment.

Film Thickness-Calculating Step

In the method for calculating a film thickness of a grain boundary oxide layer, the plurality of correlations are pre-extracted in accordance with the manufacturing conditions of the steel sheet M. For example, description is made taking, as an example, a case in which the plurality of correlations are pre-extracted in accordance with the Mn concentration in the steel sheet M; however, this does not mean that the manufacturing conditions of the steel sheet M are limited to the Mn concentration in the steel sheet M. It is to be noted that each correlation can be obtained using, for example, the method for extracting the correlation between the fluorescent X-ray intensity and the film thickness of the grain boundary oxide layer described in the first embodiment.

The plurality of correlations differ in the Mn concentration. The Mn concentration is preferably determined so as to cover, for example, a range in which manufacturing variations are taken into account, with a design reference value of the steel sheet M in the middle. In the example of the graph in FIG. 7, in addition to the case of the Mn concentration being 2.0% by mass, which is the design reference value, a case of the Mn concentration being 1.8% by mass and a case of the Mn concentration being 2.2% by mass, i.e., three correlations in total are prepared to cover the range of the manufacturing variations.

In the film thickness-calculating step, one correlation is selected from the plurality of correlations in accordance with the manufacturing conditions of the steel sheet M. In the case in which the manufacturing condition is the Mn concentration, for example, a measurement result of the base material obtained during operation may be used as the Mn concentration in the steel sheet M. By using this measurement result, the Mn concentration in the individual steel sheet M can be known, whereby a correlation of the Mn concentration close to the measurement result can be selected.

The film thickness (calculated film thickness 1a) of the grain boundary oxide layer can be easily and accurately calculated based on the fluorescent X-ray intensity by using the correlation selected.

Advantages

For example, the fluorescent X-ray intensity depends on the Mn concentration. In the case in which the manufacturing conditions of the steel sheet M are thus varied, the correlation may vary. Therefore, in the method for calculating a film thickness of a grain boundary oxide layer, by enabling selecting an appropriate correlation in accordance with the manufacturing conditions of the steel sheet M, the calculation accuracy of the film thickness can be further improved. Accordingly, even when manufacturing variations are caused in the steel sheet M, for example, the plating properties of the steel sheet M can be accurately determined from the grain boundary oxide layer.

Third Embodiment

Hereinafter, a method for determining plating properties according to a third embodiment of the present invention is described.

Method for Determining Plating Properties

The method for determining plating properties illustrated in FIG. 8 is a method for determining the plating properties of the plating properties of the steel sheet M from the grain boundary oxide layer remaining on the steel sheet M after the pickling treatment and includes: the intensity-measuring step S1 in which the fluorescent X-ray intensity of the element being contained in the steel sheet M and constituting the oxide in the grain boundary oxide layer is measured; the distance-measuring step S2 in which the measurement distance L being the distance between the steel sheet M and the measurement position of the fluorescent X-ray intensity measured in the intensity-measuring step S1 is measured; the intensity-correcting step S3 in which the fluorescent X-ray intensity measured in the intensity-measuring step S1 is corrected based on the measurement distance L; and a determining step S7 in which the plating properties of the steel sheet M are directly determined based on the fluorescent X-ray intensity corrected in the intensity-correcting step S3. The method for determining plating properties can be used in the plating property-determining step S13 illustrated in FIG. 3 of the first embodiment instead of the method for determining plating properties illustrated in FIG. 2.

The intensity-measuring step S1, the distance-measuring step S2, and the intensity-correcting step S3 are similar to the intensity-measuring step S1, the distance-measuring step S2, and the intensity-correcting step S3 in the plating property-determining step of the first embodiment illustrated in FIG. 2, and thus, detailed description thereof is omitted.

Determining Step

In the plating property-determining step of the first embodiment illustrated in FIG. 2, the film thickness of the grain boundary oxide layer is calculated to be used as the determination indicator, whereas in the plating property-determining step of the third embodiment, the plating properties are directly determined from a numerical value itself of the fluorescent X-ray intensity, without involving the film thickness of the grain boundary oxide layer.

As described above, the fluorescent X-ray intensity and the film thickness of the grain boundary oxide layer have a correlation, and thus, the plating properties can be determined when the film thickness of the grain boundary oxide layer is determined. In this case, when the fluorescent X-ray intensity is known, a determination result of the plating properties is uniquely determined, and thus, the plating properties can be defined as a function of the fluorescent X-ray intensity. In this case, the film thickness of the grain boundary oxide layer is not explicitly calculated, but a result substantially identical to a determination result obtained by calculating the film thickness of the grain boundary oxide layer can be obtained.

Advantages

In the method for determining plating properties, the fluorescent X-ray intensity of the element being contained in the steel sheet M and constituting the oxide in the grain boundary oxide layer is measured. Since the fluorescent X-ray intensity has a high correlation with the film thickness of the grain boundary oxide layer remaining on the steel sheet M after the pickling treatment, the plating properties of the steel sheet M can be directly and accurately calculated based on the fluorescent X-ray intensity.

Other Embodiments

It is to be noted that the present invention is not limited to the above embodiments.

In the above embodiments, the case in which the method for determining plating properties includes the intensity-correcting step has been described; however, the intensity-correcting step is not an essential component and may be omitted. In this case, the intensity correction portion of the plating property determination device may also be omitted.

The method for determining plating properties which does not include the intensity-correcting step preferably includes a distance-correcting step of mechanically maintaining the measurement distance. In this case, the plating property determination device is provided with a distance correction portion for mechanically maintaining the measurement distance. A method for mechanically maintaining the measurement distance may be selected as appropriate and can be exemplified by a method in which the intensity measurement portion is moved based on the measurement distance measured in the distance-measuring step. It is to be noted that in the determining step of the method for determining plating properties which does not include the intensity-correcting step, the film thickness of the grain boundary oxide layer is calculated based on: the correlation between the pre-extracted fluorescent X-ray intensity and the film thickness of the grain boundary oxide layer; and the fluorescent X-ray intensity measured in the intensity-measuring step.

A configuration which does not include the distance-measuring step but includes only the intensity-correcting step may also be employed. For example, in the intensity-measuring step, a fluorescent X-ray intensity of an Fe element may be acquired in addition to that of a target element, and the correction may be conducted based on a difference in the intensity of the Fe element.

Moreover, a method for determining plating properties which includes neither the distance-measuring step nor the intensity-correcting step also falls within the intended scope of the present invention. For example, in an environment where fluctuations in the measurement distance are ensured to be small, the film thickness of the grain boundary oxide layer can be accurately calculated without correction. In this case, both the distance measurement portion and the intensity correction portion may be omitted in the plating property determination device.

In the first embodiment, the extraction method in which the correlation between the fluorescent X-ray intensity and the film thickness of the grain boundary oxide layer is obtained from the relation between the pickling treatment time of the steel sheet sample and the decrease in mass due to the pickling treatment has been shown; however, the method for extracting the correlation is not limited thereto. For example, the film thickness of the grain boundary oxide layer may be obtained by SEM observation or the like of a cross section of each individual steel sheet sample to define the correlation.

In the first embodiment, the method in which the correlation between the fluorescent X-ray intensity and the film thickness of the grain boundary oxide layer is represented by the correlation equation has been shown; however, the correlation may be represented by another method, for example, in a lookup table form or the like.

In the above embodiments, the case in which the film thickness calculation device is provided on the downstream side of the pickling portion and the upstream side of the cold-rolling portion has been described; however, the film thickness calculation device may be provided at another position as long as the position is on the downstream side of the pickling portion. Similarly, in the method for manufacturing a plated steel sheet, the timing of the plating property-determining step is not limited to before the cold-rolling step as long as it is after the pickling step. A similar effect can be exhibited even when a cold-rolled steel sheet is measured by the intensity measurement portion of the plating property determination device.

In the first and second embodiments, the case in which the film thickness of the grain boundary oxide layer is calculated to be used as the determination indicator has been described, and in the third embodiment, the case in which the plating properties are directly determined from the numerical value itself of the fluorescent X-ray intensity has been described; however, another indicator may be used as the determination indicator.

EXAMPLES

Hereinafter, the present invention is described further in detail by way of Examples; however, the present invention is not limited to these Examples.

A steel sheet containing Mn at a content of 2.35% by mass was prepared.

To determine the correlation equation by the method for extracting the correlation between the fluorescent X-ray intensity and the film thickness of the grain boundary oxide layer described in regard to the film thickness-calculating step S4 of the first embodiment, 15 steel sheet samples with remaining grain boundary oxide layers having different film thicknesses were prepared.

Firstly, the fluorescent X-ray intensity was measured for each individual steel sheet sample. As the fluorescent X-ray analyzer, Simultix-14, manufactured by Rigaku, was used.

Next, the relation between the pickling treatment time of each individual steel sheet sample and the decrease in mass per unit area due to the pickling treatment was obtained. An example thereof is shown in FIG. 9. As shown in FIG. 9, with regard to each individual steel sheet sample, a polyline was obtained as the relation between the pickling treatment time and the decrease in mass due to the pickling treatment. The converted film thickness of the grain boundary oxide layer of each individual steel sheet sample was calculated by dividing the decrease in mass at the above breakpoint by the iron density of 7.87 g/cm$^3$.

FIG. 10 shows a graph in which with regard to each individual steel sheet sample, the relation between the fluorescent X-ray intensity and the film thickness of the grain boundary oxide layer is plotted. As seen from the graph in FIG. 10, it is found that there is a negative correlation between the fluorescent X-ray intensity and the film thickness of the grain boundary oxide layer.

A relation between a fluorescent X-ray intensity x (kcps) and a film thickness y ($\mu$m) of the grain boundary oxide layer was calculated by the least squares method using linear approximation to obtain the following equation 4.

$$y = -0.0832x + 28.216 \qquad 4$$

FIG. 11 shows, with regard to the 15 steel sheet samples, a correlation between actually measured values and the film thickness of the grain boundary oxide layer calculated according to the method for determining plating properties of the present invention by using the above correlation equation 4. It is to be noted that the actually measured values are converted film thickness values calculated in such a manner that for each individual steel sheet sample, the decrease in mass was calculated from the breakpoint on the above-mentioned polyline of the pickling treatment time and the decrease in mass due to the pickling treatment, and the decrease in mass was divided by the iron density of 7.87 g/cm$^3$.

It can be seen from the results in FIG. 11 that the measurement can be conducted within a range of greater than or equal to 2 $\mu$m and less than or equal to 8 $\mu$m, which is a measurement range, with high accuracy such that a standard deviation $\sigma$ = 0.88 $\mu$m. Accordingly, it can be said that the plating properties of the steel sheet can be determined by using, as the determination indicator, the film thickness of the grain boundary oxide layer calculated based on the fluorescent X-ray intensity.

[INDUSTRIAL APPLICABILITY]

According to the method for calculating a film thickness of a grain boundary oxide layer and the film thickness calculation device of the present invention, the film thickness of the grain boundary oxide layer remaining on the steel sheet after the pickling treatment can be accurately calculated. According to the method for determining plating properties of the present invention, the plating properties of the steel sheet can be accurately determined from the grain boundary oxide layer remaining on the steel sheet after the pickling treatment. Furthermore, the method for manufacturing a plated steel sheet of the present invention includes the method for determining plating properties which enables accurately determining the plating properties of the steel sheet from the grain boundary oxide layer, thereby increasing the manufacturing efficiency.

[EXPLANATION OF THE REFERENCE SYMBOLS]

| | |
|---|---|
| 1, 3 | Film thickness calculation device |
| 2 | Plating property determination device |
| 1a | Calculated film thickness |
| 10 | Intensity measurement portion |
| 20 | Distance measurement portion |
| 30 | Intensity correction portion |
| 40, 41 | Film thickness calculation portion |
| 50 | Determination portion |
| 60 | Storage portion |
| 100 | Plated steel sheet-manufacturing device |

| | |
|---|---|
| 110 | Hot-rolling portion |
| 120 | Pickling portion |
| 130 | Cold-rolling portion |
| M | Steel sheet |
| C1 | Cast coil |
| C2 | Uncooled hot-rolled coil |
| C3 | Cooled hot-rolled coil |
| C4 | Cold-rolled coil |
| L | Measurement distance |
| R | Sheet passing direction |

**Claims**

1. A method for calculating a film thickness of a grain boundary oxide layer remaining on a steel sheet after a pickling treatment, the method comprising:

    intensity-measuring, wherein a fluorescent X-ray intensity of an element being comprised in the steel sheet and constituting an oxide in the grain boundary oxide layer is measured; and
    film thickness-calculating, wherein a film thickness of the grain boundary oxide layer is calculated based on: a correlation between a pre-extracted fluorescent X-ray intensity and the film thickness of the grain boundary oxide layer; and the fluorescent X-ray intensity measured in the intensity-measuring.

2. The method for calculating a film thickness of a grain boundary oxide layer according to claim 1, wherein

    a plurality of correlations are pre-extracted in accordance with manufacturing conditions of the steel sheet, and
    in the film thickness-calculating, one correlation is selected from the plurality of correlations in accordance with the manufacturing conditions of the steel sheet.

3. The method for calculating a film thickness of a grain boundary oxide layer according to claim 1 or 2, the method further comprising:

    distance-measuring, wherein a measurement distance is measured, the measurement distance being a distance between the steel sheet and a measurement position of the fluorescent X-ray intensity measured in the intensity-measuring; and
    intensity-correcting, wherein the fluorescent X-ray intensity measured in the intensity-measuring is corrected based on the measurement distance,
    wherein in the film thickness-calculating, the fluorescent X-ray intensity corrected in the intensity-correcting is used instead of the fluorescent X-ray intensity measured in the intensity-measuring.

4. The method for calculating a film thickness of a grain boundary oxide layer according to claim 1 or 2, wherein

    an acid used in the pickling treatment is hydrochloric acid, and
    the element is Mn.

5. A method for determining plating properties of a steel sheet from a grain boundary oxide layer remaining on the steel sheet after a pickling treatment, the method comprising:

    calculating a film thickness of the grain boundary oxide layer; and
    determining plating properties of the steel sheet by using, as a determination indicator, the film thickness of the grain boundary oxide layer calculated in the calculating,
    wherein the method for calculating a film thickness of a grain boundary oxide layer according to claim 1 is used in the calculating.

6. A method for determining plating properties of a steel sheet from a grain boundary oxide layer remaining on the steel sheet after a pickling treatment, the method comprising:

    intensity-measuring, wherein a fluorescent X-ray intensity of an element being comprised in the steel sheet and constituting an oxide in the grain boundary oxide layer is measured; and
determining, wherein plating properties of the steel sheet are directly determined based on the fluorescent X-ray intensity measured in the intensity-measuring.

**7.** A method for manufacturing a plated steel sheet, the method comprising:

hot-rolling, wherein a steel sheet serving as a base material is hot-rolled;
pickling, wherein an oxide scale on a surface of the steel sheet after the hot-rolling is removed by an acid; and
plating property-determining, wherein plating properties of the steel sheet are determined from a grain boundary oxide layer remaining on the steel sheet after the pickling,
wherein the method for determining plating properties according to claim 5 or 6 is used in the plating property-determining.

**8.** A film thickness calculation device for measuring a film thickness of a grain boundary oxide layer remaining on a steel sheet after a pickling treatment, the film thickness calculation device comprising:

an intensity measurement portion for measuring a fluorescent X-ray intensity of an element being comprised in the steel sheet and constituting an oxide in the grain boundary oxide layer;
a distance measurement portion for measuring a measurement distance being a distance between the steel sheet and a measurement position of the fluorescent X-ray intensity of the intensity measurement portion;
an intensity correction portion for correcting, based on the measurement distance, the fluorescent X-ray intensity measured by the intensity measurement portion; and
a film thickness calculation portion for calculating a film thickness of the grain boundary oxide layer based on: a correlation between a pre-extracted fluorescent X-ray intensity and the film thickness of the grain boundary oxide layer; and the fluorescent X-ray intensity corrected by the intensity correction portion.

**9.** The film thickness calculation device according to claim 8, further comprising a storage portion for storing a plurality of correlations extracted in accordance with manufacturing conditions of the steel sheet,
wherein the film thickness calculation portion comprises a function of selecting one correlation from the plurality of correlations in accordance with the manufacturing conditions of the steel sheet.

FIG. 1

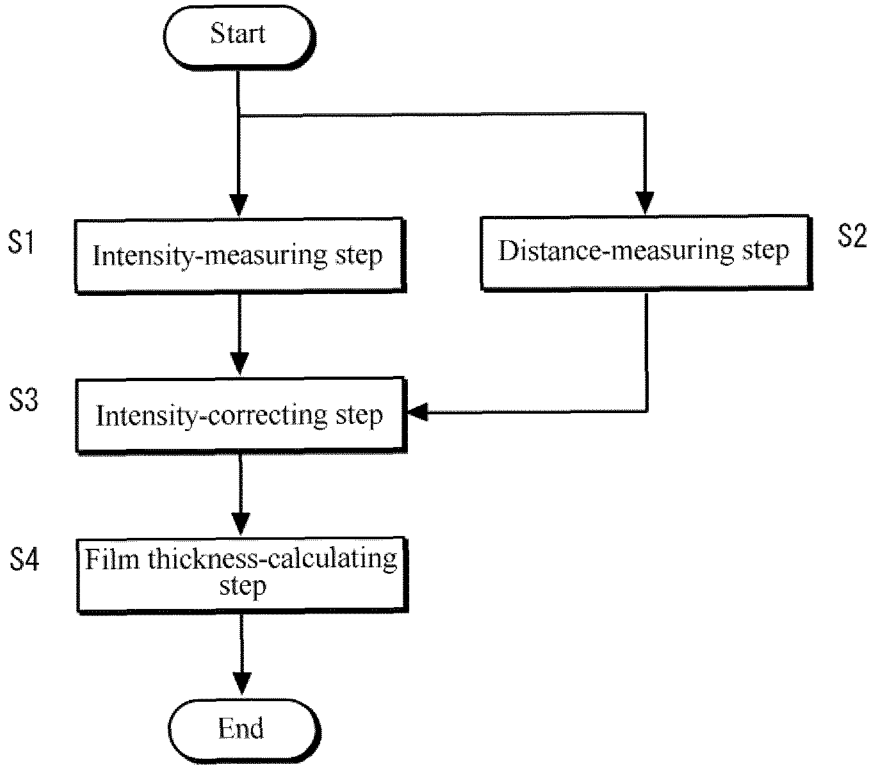
Start
S1
Intensity-measuring step
S2
Distance-measuring step
S3
Intensity-correcting step
S4
Film thickness-calculating step
End

FIG. 2

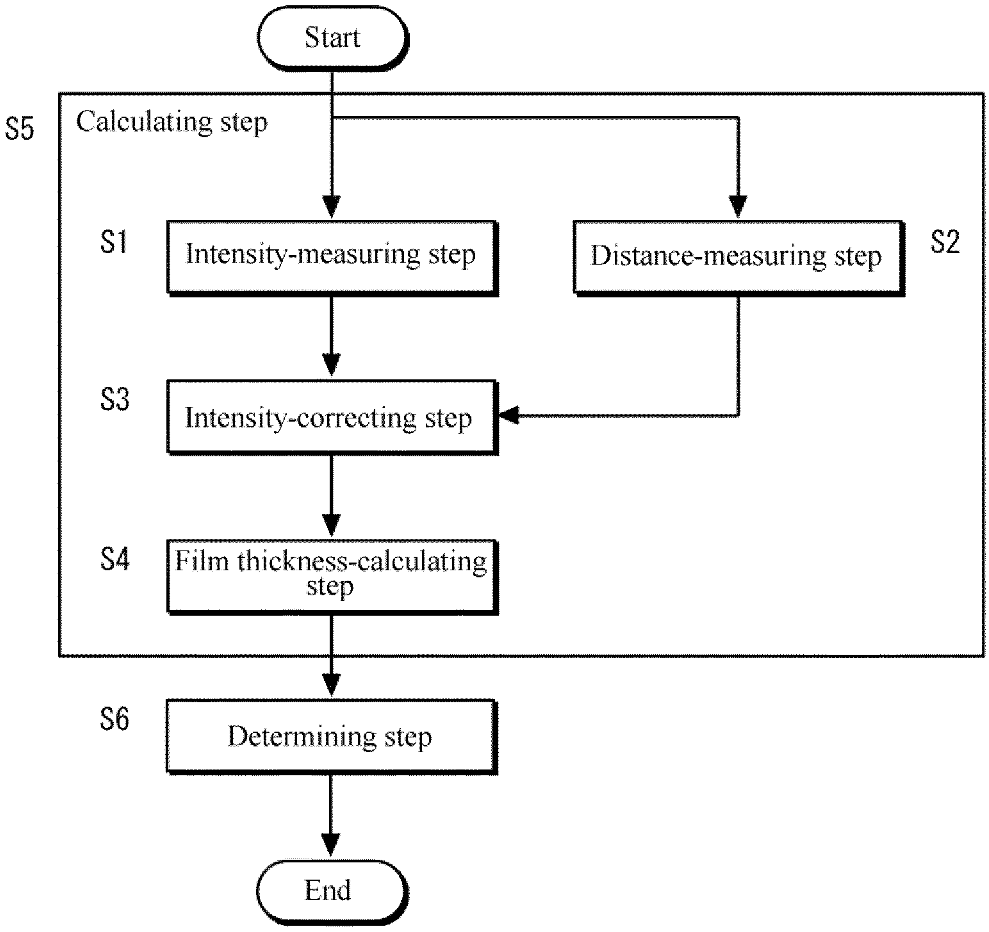
Start
S5
Calculating step
S1
Intensity-measuring step
Distance-measuring step
S2
S3
Intensity-correcting step
S4
Film thickness-calculating step
S6
Determining step
End

FIG. 3

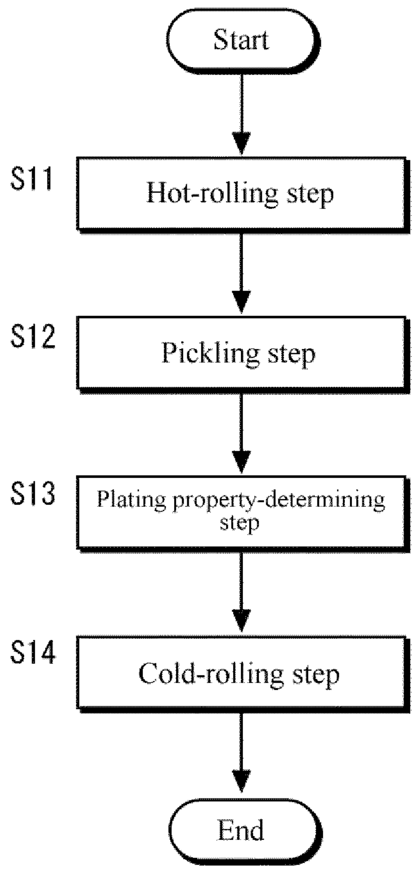
Start
S11
Hot-rolling step
S12
Pickling step
S13
Plating property-determining step
S14
Cold-rolling step
End

FIG. 4

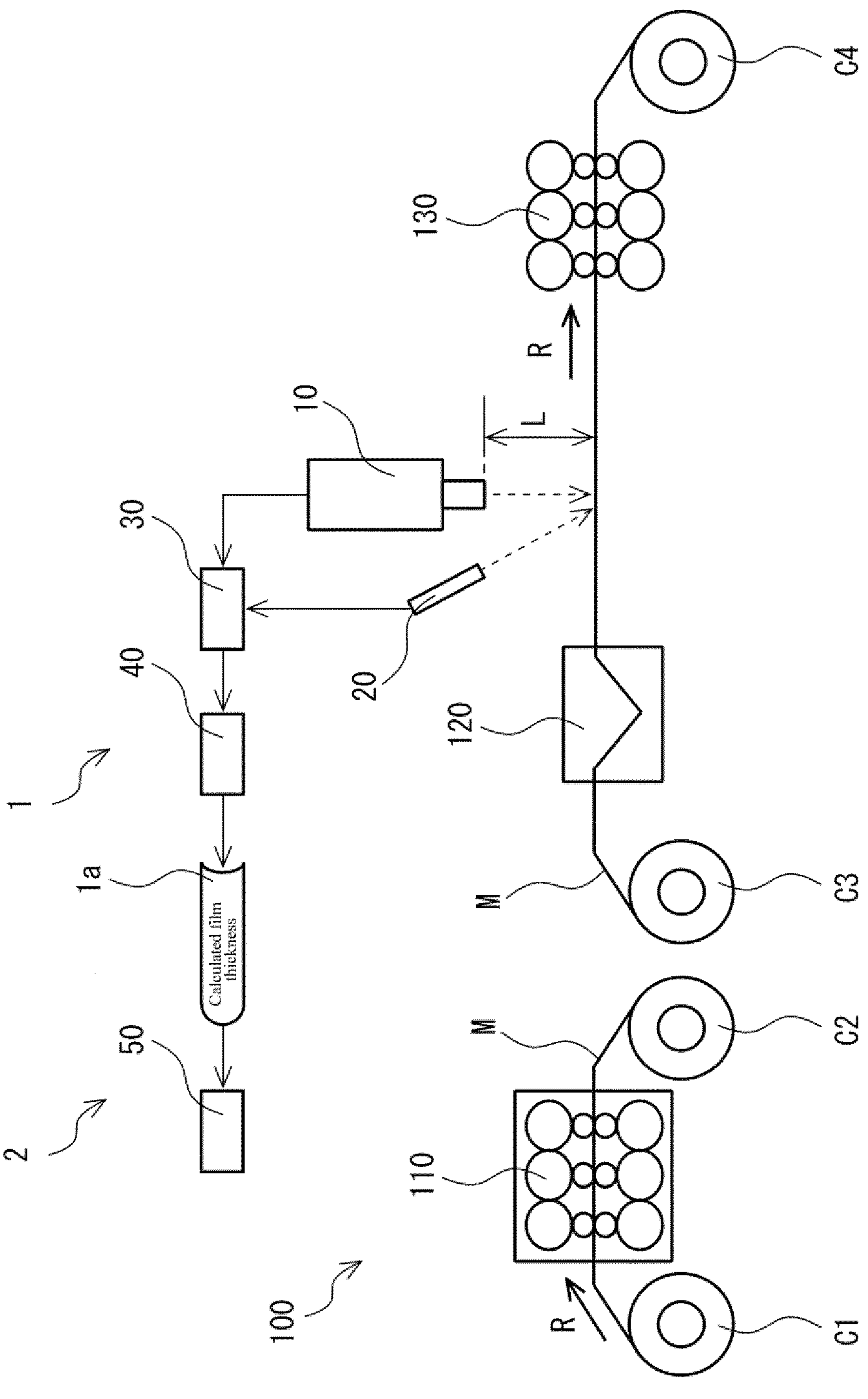
C4
130
R
10
L
30
20
40
120
1
1a
Calculated film thickness
M
C3
C2
M
50
2
110
100
R
C1

FIG. 5

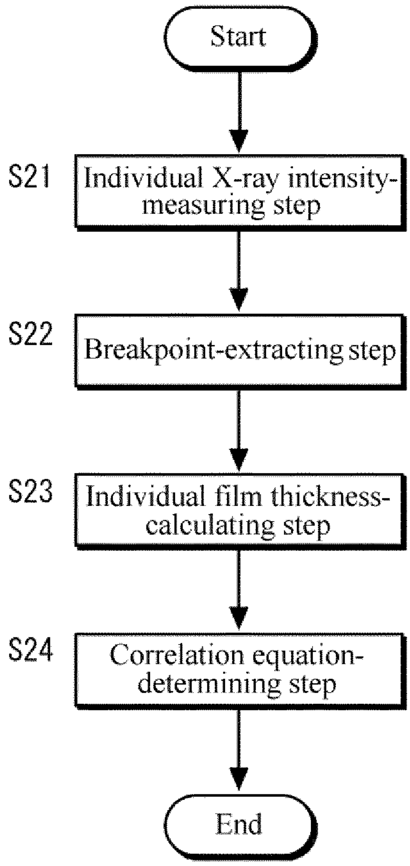
Start
S21
Individual X-ray intensity-measuring step
S22
Breakpoint-extracting step
S23
Individual film thickness-calculating step
S24
Correlation equation-determining step
End

FIG. 6

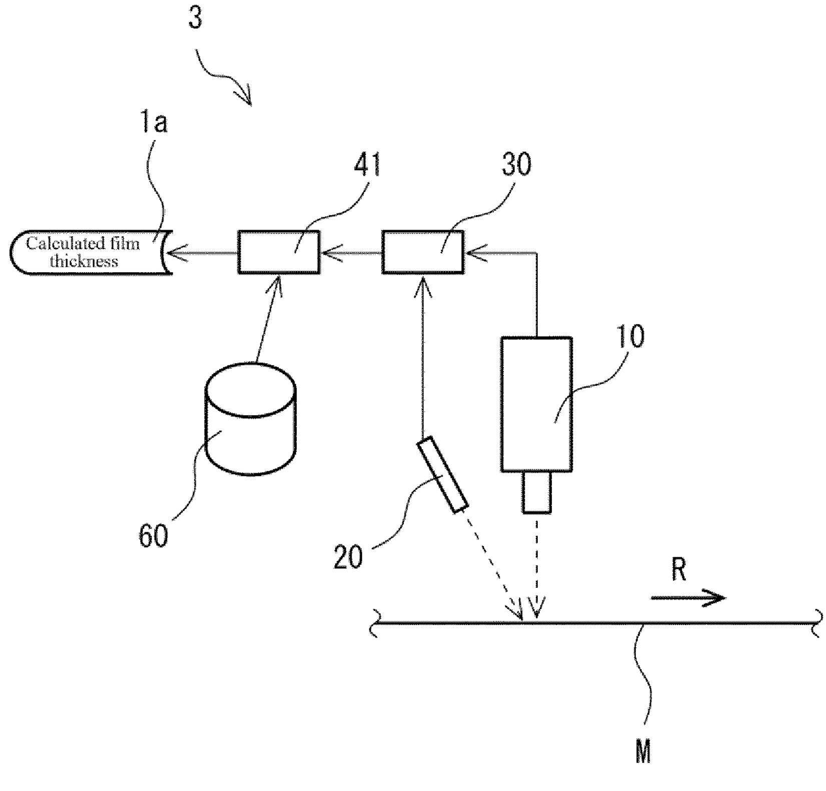

FIG. 7

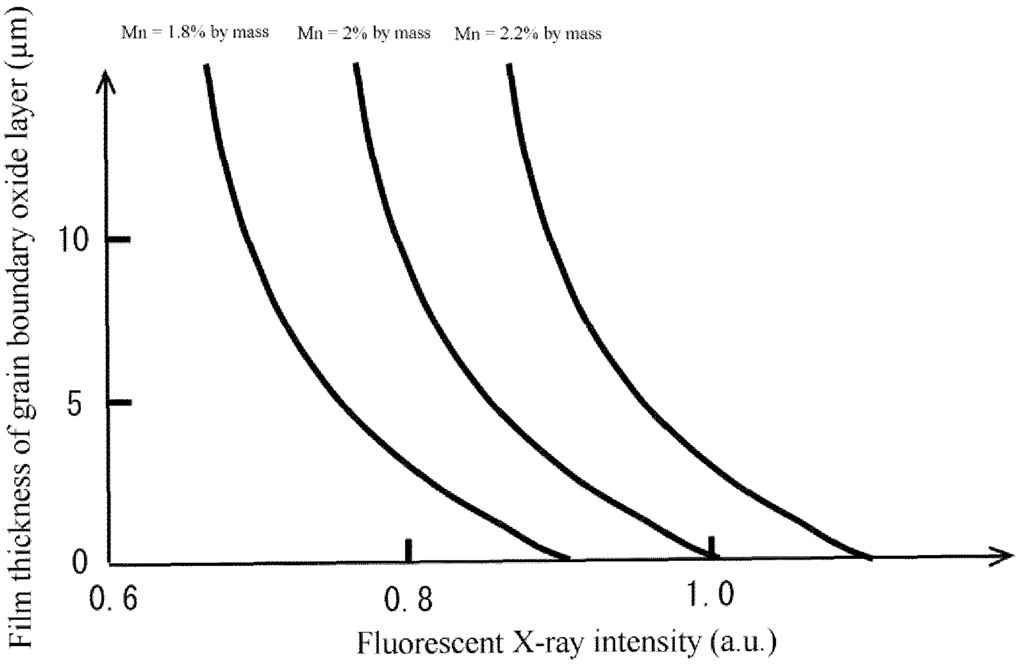
Mn = 1.8% by mass
Mn = 2% by mass
Mn = 2.2% by mass
Film thickness of grain boundary oxide layer (µm)
10
5
0
0. 6
0. 8
1. 0
Fluorescent X-ray intensity (a.u.)

FIG. 8

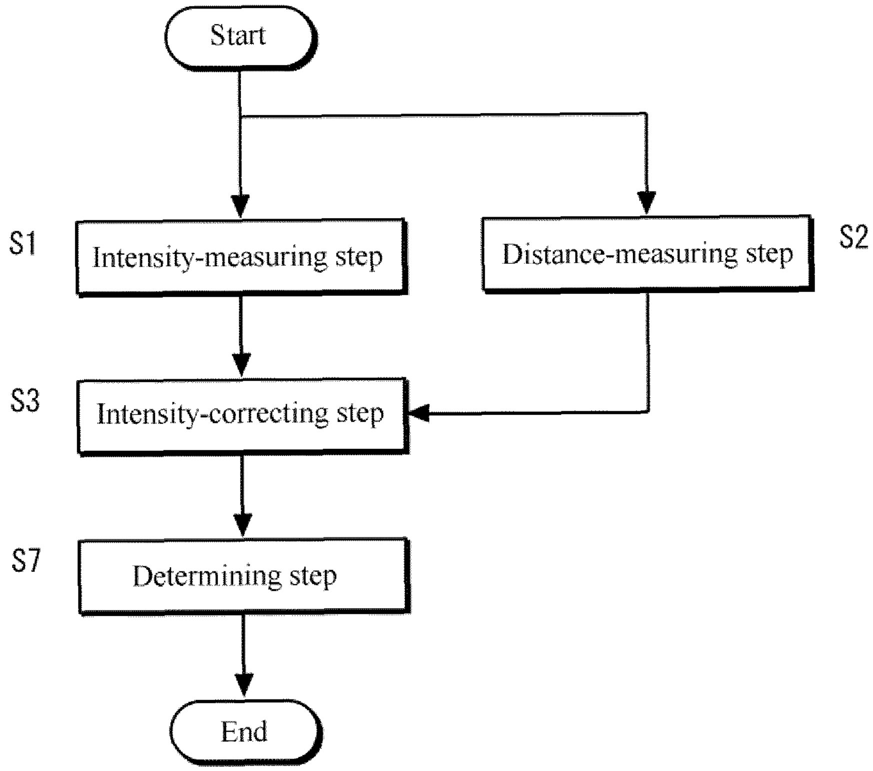
Start
S1
Intensity-measuring step
Distance-measuring step
S2
S3
Intensity-correcting step
S7
Determining step
End

FIG. 9

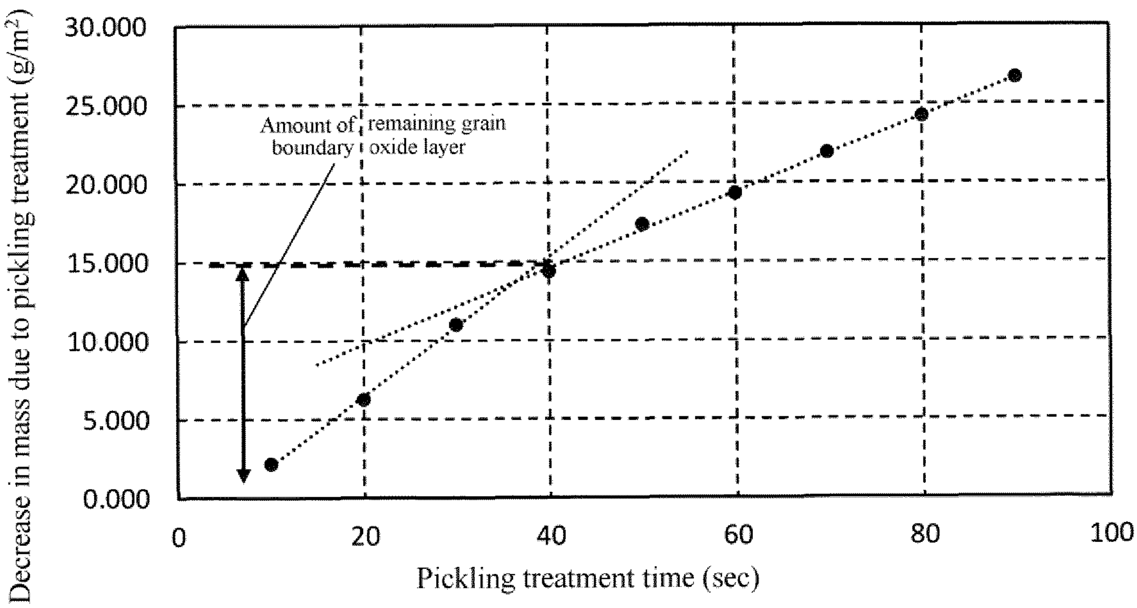
Decrease in mass due to pickling treatment (g/m2)
30.000
25.000
20.000
15.000
10.000
5.000
0.000
0
20
40
60
80
100
Pickling treatment time (sec)
Amount of remaining grain boundary oxide layer

FIG. 10

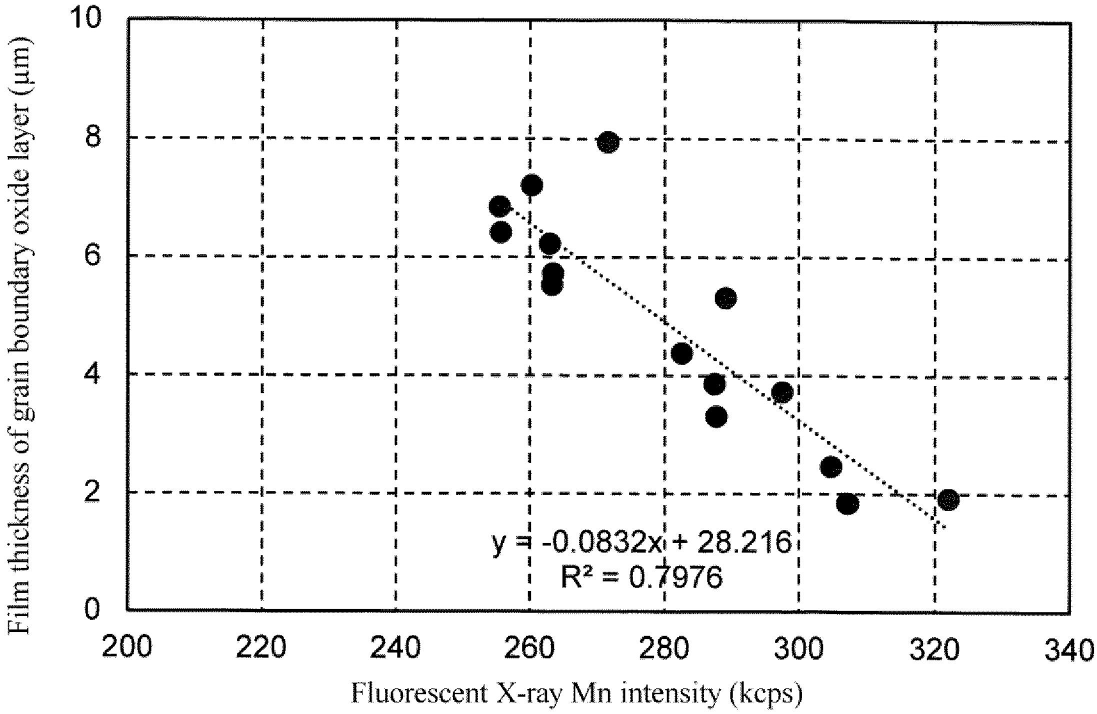

Film thickness of grain boundary oxide layer (μm)
10
8
6
4
2
0
200
220
240
260
280
300
320
340
Fluorescent X-ray Mn intensity (kcps)
y = -0.0832x + 28.216
R² = 0.7976

FIG. 11

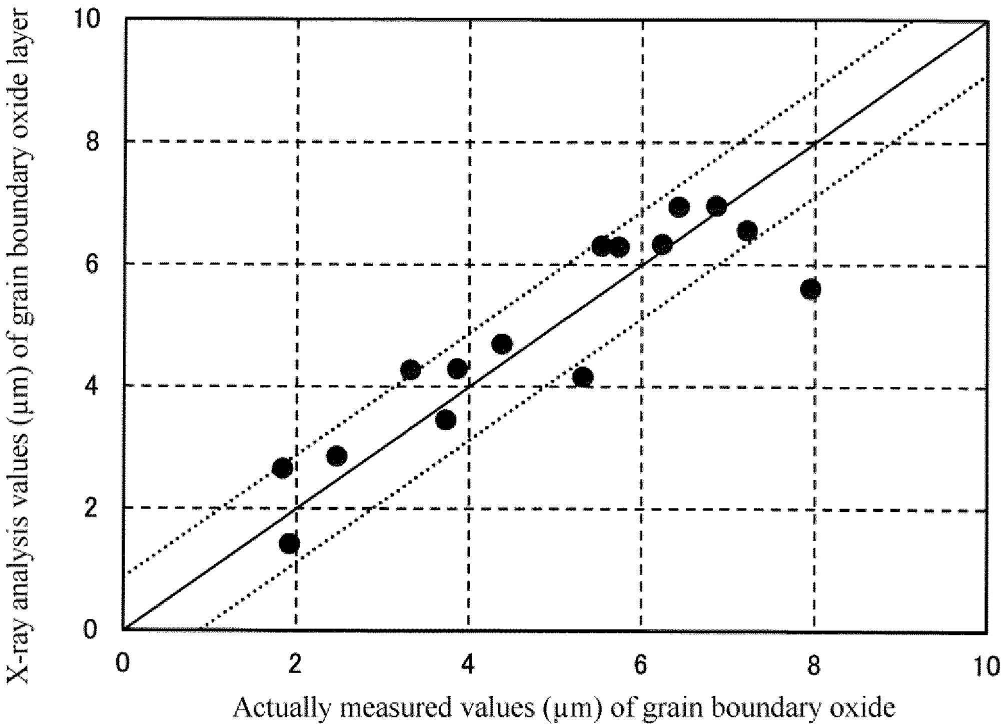
X-ray analysis values (µm) of grain boundary oxide layer
10
8
6
4
2
0
0
2
4
6
8
10
Actually measured values (µm) of grain boundary oxide

**INTERNATIONAL SEARCH REPORT**

International application No. **PCT/JP2022/039864**

**A. CLASSIFICATION OF SUBJECT MATTER**

***C23C 2/02***(2006.01)i; ***G01N 23/223***(2006.01)i; ***G01N 33/2028***(2019.01)i
FI: G01N23/223; C23C2/02; G01N33/2028

According to International Patent Classification (IPC) or to both national classification and IPC

**B. FIELDS SEARCHED**

Minimum documentation searched (classification system followed by classification symbols)

G01N23/00-23/2276

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 6-88794 A (SUMITOMO METAL IND LTD) 29 March 1994 (1994-03-29)<br>paragraphs [0002]-[0043], fig. 1-2 | 1-2, 4<br>3, 5-9 |
| Y | JP 2018-63185 A (NIPPON STEEL & SUMITOMO METAL CORP) 19 April 2018 (2018-04-19)<br>paragraph [0058] | 3, 8-9 |
| Y | JP 10-282020 A (KAWASAKI STEEL CORP) 23 October 1998 (1998-10-23)<br>paragraphs [0002]-[0003] | 5-7 |
| A | KR 10-2016-0079250 A (POSCO) 06 July 2016 (2016-07-06)<br>entire text, all drawings | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 November 2022** | **06 December 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/039864**

| Patent document cited in search report | Publication date (day/month/year) | Patent family member(s) | Publication date (day/month/year) |
|---|---|---|---|
| JP 6-88794 A | 29 March 1994 | (Family: none) | |
| JP 2018-63185 A | 19 April 2018 | (Family: none) | |
| JP 10-282020 A | 23 October 1998 | (Family: none) | |
| KR 10-2016-0079250 A | 06 July 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001272360 A